# EUROPEAN PATENT SPECIFICATION

(11) **EP 1 986 490 B1**
(45) Date of publication and mention of the grant of the patent: **23.02.2011**
(21) Application number: 04721431.7
(22) Date of filing: 18.03.2004
(51) Int. Cl.: A01K 67/027, C12N 15/12

(54) **MODEL FOR MUSCULAR DYSTROPHY AND CARDIOMYOPATHY**
MODELL FÜR MUSKELDYSTROPHIE UND KARDIOMYOPATHIE
MODELE POUR DYSTROPHIE MUSCULAIRE ET CARDIOMYOPATHIE

(30) Priority: 19.03.2003 AU 2003901269
(43) Date of publication of application: 05.11.2008
(73) Proprietor: The Victor Chang Cardiac Research Institute Limited, Darlinghurst, New South Wales 2010 (AU)
(72) Inventor: CURRIE, Peter, Ashbury, NSW 2193 (AU); BASSETT, David, Blaydon Gateshead NE21 4ND (GB)
(74) Representative: Almond-Martin, Carol
(86) International application number: PCT/AU2004/000337
(87) International publication number: WO 2004/082374

(56) References cited:
- BROWN LOUISE A ET AL: "Molecular characterization of a zebrafish TCF ETS-domain transcription factor" ONCOGENE, vol. 18, no. 56, 23 December 1999 (1999-12-23), pages 7985-7993, XP002386009 ISSN: 0950-9232
- BOLANOS-JIMENEZ FRANCISCO ET AL: "Dystrophin and Dp71, two products of the DMD gene, show a different pattern of expression during embryonic development in zebrafish" MECHANISMS OF DEVELOPMENT, vol. 102, no. 1-2, April 2001 (2001-04), pages 239-241, XP002386010 ISSN: 0925-4773
- GUYON J.R. ET AL.: 'The dystropihin associated protein complex in zebrafish' HUMAN MOLECULAR GENETICS vol. 12, no. 6, March 2003, pages 601 - 615, XP002999091
- PARSONS M.J. ET AL.: 'Removal of dystroglycan causes severe muscular dystrophy in zebrafish embryos' DEVELOPMENT AND DISEASE vol. 129, 2002, pages 3505 - 3512, XP002999092
- CHAMBERS S.P. ET AL.: 'Dystrophin in adult zebrafish muscle' BIOCHEMICAL AND BIOPHYSICAL RESEARCH COMMUNICATIONS vol. 286, no. 3, 21 August 2001, pages 478 - 483, XP002999093
- BOLANOS-JIMENEZ F. ET AL.: 'Molecular cloning and characterization of dystrophin and Dp71, two products of the Duchene muscular dystrophy gene, in zebrafish' GENE vol. 274, no. 1-2, 22 August 2001, pages 217 - 226, XP004311068

## Description

### Technical Field

The present invention relates to zebrafish models for studying muscular dystrophy and cardiomyopathy. The invention is also suitable for screening agents which may have an affect on the clinical manifestations of muscular dystrophy or cardiomyopathy.

### Background Art

Genetic lesions within the structural muscle protein Dystrophin lead to the onset of the fatal muscle wasting diseases, Becker (BMD) and Duchenne (DMD) muscular dystrophies, as well as other dystrophinopathies in humans. Dystrophin is associated with a number of glycoproteins which span the sarcolemma of skeletal and cardiac muscle to form the Dystrophin-glycoprotein complex. Although the exact role that this complex plays in muscle physiology is not fully understood, it is thought that it provides an important structural link between the cytoskeleton and the extracellular matrix of muscle cells. This connection is theorised to be critical in regulating stresses that develop during muscle contraction. The lack of Dystrophin in DMD patients results in a reduction in all of the components of the Dystrophin-glycoprotein complex and a breakage in this transmembrane linkage. A weakening of the muscle membrane results, which consequently manifests itself in the pathological features of DMD. A number of diagnostic features of DMD become evident including cycles of muscle cell necrosis and regeneration, and elevated levels of muscle creatine kinase. At an advanced stage of the disease, muscle is replaced by connective tissue and fat. Consequently, DMD afflicted individuals suffer from progressive muscular wasting and usually die before the age of 20 from respiratory or cardiac failure.

DMD, BMD and X-linked dilative cardiomyopathy (XLDCM) all result from mutations within the human dystrophin gene. The heart is also affected to various degrees, depending on the stage of the disease and the type of the mutation. The pathology of cardiac disease in dystrophinopathies is the replacement of myocardium by connective tissue or fat. In DMD/BMD with the left ventricular posteriobasal and lateral walls being most extensively affected, sparing the right ventricle and the atrium. The degree and dynamics of cardiac diseases vary among the three entities. In DMD/BMD, heart disease usually remains subclinical in the early stages of the disease. Initially, echocardiography is normal or shows regional wall motion abnormalities in areas of fibrosis. With spreading of fibrosis, left ventricular dysfunction and ventricular arrhythmias additionally occur. In the final stages of the disease, systolic function may lead to heart failure and sudden death. Subclinical or clinical cardiac disease is present in about 90% of the DMD/BMD patients but is the cause of death In only 20% of the DMD and 50% of the BMD patients. XLDCM is a rapidly progressive, almost exclusively myocardial disorder, starting in teenage males as heart failure due to dilative cardiomyopathy leading to death from intractable heart failure within 1-2 years after diagnosis.

Research into the events that determine the human dystrophic pathology has benefited from the generation of mice mutant In the mouse *Dystrophin* gene *mdx.* However, despite mdx mutant mice demonstrating a number of phenotypic hall marks of muscular dystrophy early in their development, adult *mdx* mice show little muscle weakness and heart pathology and live near normal life spans, a consequence, perhaps, of the substantial muscle regeneration evident within these mice post weaning. The lack of similarity between the mouse model and the human disease has hampered research into the function of dystrophin in maintaining muscle integrity. Other large animal models such as dystrophic dogs have also been identified, but their large size and generation times make them highly unsuitable laboratory models:

Guyon et al., 2003 (Human Molecular Genetics, 2003, 12(6):601-605) discloses the use of a "morpholino" oligo to knockdown of the zebrafish dystrophin gene. There is no teaching in this publication of a zebrafish strain having a mutation in the dystrophin gene.

The present inventors have identified genes and mutations In zebrafish which correspond to mammalian genes which have been implicated in muscular dystrophy and cardiomyopathy and have developed a fish model for muscular dystrophy and cardiomyopathy. The model is highly penetrant and presents with many of the hall marks of the human disease. In short, the zebrafish laboratory dystrophic model overcomes the major short coming of mammalian models in terms of disease penetrance and manipulability of a laboratory model system

### Disclosure of Invention

In a first aspect, the present invention provides isolated zebrafish genetic strain having a dystrophin mutant phenotype resulting from a mutation within the zebrafish dystrophin gene, wherein the zebrafish has a sapje (sap) phenotype as defined herein.

Large-scale mutatgenic screens of the zebrafish genome have identified numerous mutations that disrupt differentiation and maintenance of skeletal muscle within the zebrafish embryo. Mutants possess phenotypes that range from a failure of myoblasts to elongate and fuse into a mulinucleate muscle fibres to those that exhibit muscle degeneration reminiscent of human muscular dystrophies. Homozygous mutants of this latter class form myofibrils normally but are lost focally or globally, depending on the loci Involved, during early larval life. One member of the zebrafish dystrophic mutant class has its phenotype resulting from mutations within the zebrafish dystrophin orthologue. A detailed characterisation of the phenotype that arises as a consequence of the loss of dystrophin expression within the embryonic and larval myotomes of zebrafish has been carried out. This analysis points to the critical and novels roles that the dystrophin and its associated-glycoprotein complex plays in the ontogeny of zebrafish muscle. The function of dystrophin in teleost and mammalian muscle fibre biogenesis has been compared. This analysis represents the first identification of a zebrafish model of a human muscular dystrophy/cardiomyopathy and has applications to the study of the human dystrophic condition, both in cardiomyopathy and muscular dystrophy.

Preferred mutants are sapje tm90c, sapje tj7, sapje ta222a, being the currently identified mutant alleles within the zebrafish dystrophin gene, and any combinations or other alleles that are generated being defined as mutations within the zebrafish dystrophin gene or any zebrafish strain resulting from a mutation within the zebrafish dystrophin gene.

As the present inventors have developed zebrafish genetic strains having a dystrophin mutant phenotype resulting from one or more mutations within the zebrafish dystrophin gene, it will be appreciated that other mutations of the zebrafish dystrophin gene would be contemplated from the teaching of the present invention.

It will be appreciated that further mutants, progeny, fry, gametes are also included.

In a second aspect, the present invention provides a fish model of mammalian muscular dystrophy comprising an isolated zebrafish according to the first aspect of the present invention.

Preferably, the model is of human muscular dystrophy.

In a third aspect, the present invention provides a method for screening agents having potential activity on muscular dystrophy comprising:
(a) providing a fish model according to the second aspect of the present invention;
(b) exposing the zebrafish to an agent; and
(c) determining any effect of the agent on a genetic or physical characteristic of the zebrafish or its progeny.

The agent may be a drug candidate, chemical, nucleic acid and the like.

The agent may be administered by direct dilution in raising media.

The effect may be determined by any visual or light microscopic technique including those that utilise transgenic reporter gene expression to monitor muscle integrity. They include, but not limited to, simple optical inspection of living muscle tissue, birefringency of muscle tissue using polarised light, the use of Green fluorescent protein (GFP) transgenic lines driven by muscle specific promoter(s), the use of immunohistochemistry, using antibodies directed against muscle specific epitopes and *in situ* hybridisation for muscle specific gene expression.

The present invention also discloses an agent determined to have activity on muscular dystrophy or cardiomyopathy by the method according to the third aspect of the present invention.

The present invention also discloses a method for monitoring or testing the effect of an agent having activity on muscular dystrophy or cardiomyopathy comprising:
(a) providing a fish model according to the second aspect of the present invention;
(b) exposing the zebrafish to the agent; and
(c) monitoring the effect of the agent on a genetic or physical characteristic of the zebrafish or its progeny.

The present inventors have identified a class of zebrafish mutations as candidates for mutation in human muscular dystrophy disease genes. The molecular lesion in one of these mutations, *sapje* has been identified. The *sapje* phenotype resulted from mutations within the dystrophin gene, human mutations in which result in Becker's and Duchenne Muscular Dystrophy, the most common forms of muscular dystrophy as well as cardiomyopathy.

The present inventors have established a formal link between the phenotype of this particular class of zebrafish mutations and human muscular dystrophies and cardiomyopathy. The phenotypes of these mutations have been characterised in detail, including that of the *sapje* mutation analysed in the results section below. These mutations exhibit muscle weakness in a similar manner to that described to occur in human patients. The phenotype of *sapje* (the zebrafish dystrophin mutation) has been characterised in the most detail. This phenotype results from a loss of membrane integrity on the inner membrane region, consistent with a failure of dystrophin to link from the actin cytoskeleton to the sarcolemma. This results in membrane tearing in an identical fashion to human muscle fibres deficient in dystrophin. The mutation has been mapped on the zebrafish genetic map, establishing linkage to dystrophin, assembled full-length zebrafish and fugu (pufferfish) dystrophin genes, and performed mutation detection on DNA derived from the homozygous *sapje* mutants. Stop codons have been identified within the zebrafish dystrophin open reading frame in *sapje* homozygotes. Antisense oligo-mediated knockdown of dystrophin results in an identical phenotype to that produced by the *sapje* mutation. It has also been found that the *sapje* phenotype results from a mutation in the zebrafish *dystrophin* gene.

The highly penetrant nature of the zebrafish dystrophin mutant phenotype more closely mirrors the human phenotype than that present in the MDX mouse model, suggesting it will be a very useful tool. A number of attributes of zebrafish biology and development lend themselves to the implementation of a high through out screening rationales for genetic and pharmacological modifiers of the dystrophic phenotype. External fertilisation, high fecundity, optical transparency and small size of the embryos will allow us to directly screen for chemicals or second site mutations that modulate the dystrophic phenotype. These findings would form the basis of drug design for treatment of the human dystrophic condition.

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed in Australia before the priority date of each claim of this application.

In order that the present invention may be more clearly understood, preferred forms will be described with reference to the following drawings and examples.

### Brief Description of the Drawings

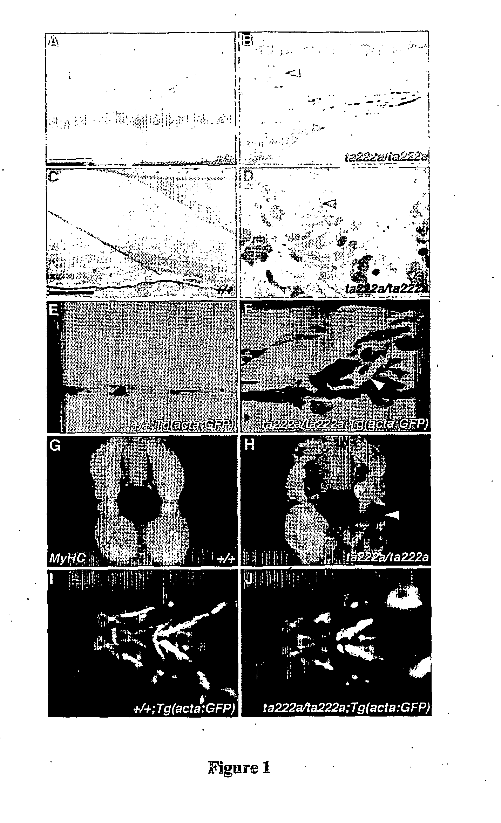

Figure 1 shows *sap* mutants develop lesions in skeletal muscle where fibres detach from myosepta and retract. Fibres in wild type span the entire somite between myosepta (arrowheads in A; lateral views, 48 hours post-fertilisation), whereas lesions within *sap* somites are evident as cell free spaces (arrowheads in **B**). Toluidene blue histology revealed detached, retracted fibres in association with lesions in *sap* (arrowhead in **D;** parasagittal sections, 72 hours post-fertilisation) but not in wild type **(C).** Reconstruction of somites in 3D using confocal microscopy of fluorescence from the *Tg(acta:GFP)* transgene reveals extensive fibre loss in *sap* (arrowhead in **F)** but not wild type (E). Anti-MyHC (green) reveals that while differentiation is normal in both wild type (G) and *sap* **(H),** lesions in *sap* mutant somites lack contractile apparatus (arrowhead). Examination of head musculature using fluorescence from the *Tg(acta:GFP)* transgene showed that these muscles are unaffected in *sap* **(J)** compared to wild type **(I)**.

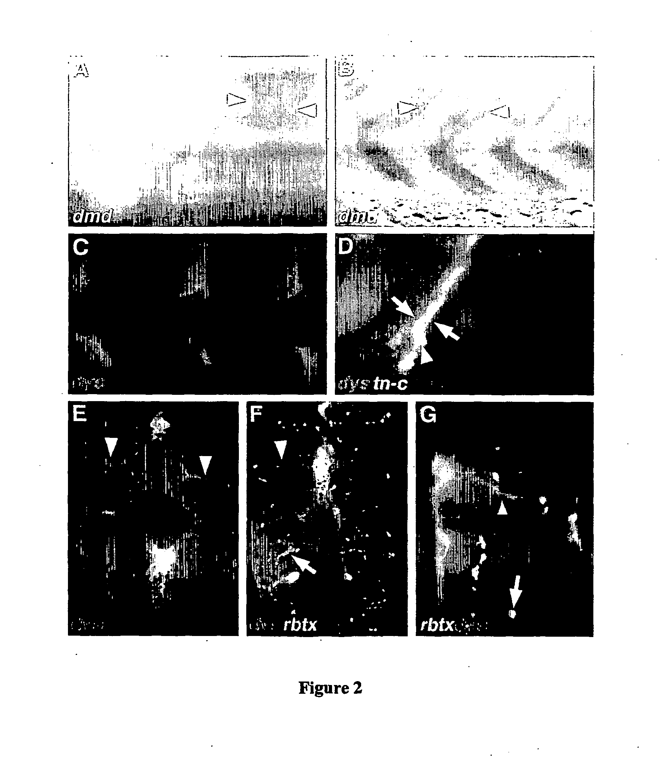

Figure 2 shows dystrophin is associated with the wild type embryonic muscle attachments. *dystrophin* mRNA (*dmd*) localises intracellularly to wild type somite boundaries both before (19 hours post-fertilisation, **A)** and after (27 hours post-fertilisation, **B)** muscle fibre differentiation (arrowheads, lateral views). At 19 hours post-fertilisation, a crescent of mRNA is present at one side of undifferentiated cells that abut somite boundaries. Dystrophin protein (dys) is localised embryonically to fibre ends at somite boundaries, and at NMJs but not at the sarcolemma **(C,** horizontal section, 72 hours post-fertilisation). Dystrophin (green) in fibre ends sandwiches the ECM of the vertical myoseptum at somite boundaries, which contain tenascin-C (tn-c, red; arrowhead in **D**, horizontal section, 72 hours post-fertilisation). Dystrophin localises to fibre ends and NMJs but not to the sarcolemma embryonically (arrowheads in **E**, transverse section, 72 hours post-fertilisation. Dystrophin is detectable at muscle attachments (arrowheads in **E-G**), but triple labelling using anti-dystrophin (green), Alexa594-α-Bungarotoxin to label NMJs (rbtx, red), and DAPI (blue) reveals that dystrophin within the myotome is at NMJs, co-localising with rbtx to producing an overlapping yellow signal (arrows in **F, G**; horizontal sections 72 hours post-fertilisation).

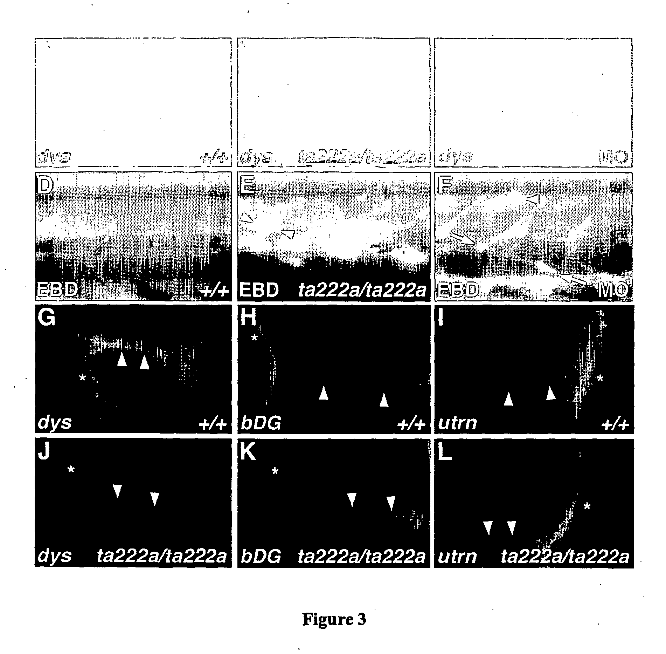

Figure 3 shows *sap*^{ta222a} is a mutation of *dmd* that causes fibre detachment and is phenocopied by knocking down *dmd* function. Dystrophin C-terminal immunoreactivity is localised to somite boundaries in wild type (A, 27 hours post-fertilisation, lateral views) but lacking in both *sap* (**B**) and dystrophin-morphant embryos (**C**). Evans blue dye (**EBD,** red), does not appear in wild type somites (**D**), but labels fibres in both *sap* (**E**) and dystrophin-morphant embryos (**F**). Labelled fibres are visible that have both detached and retracted (arrowheads) or still span a somite and show a retraction zone (between arrows in **F,** lateral views, 72 hours post-fertilisation). By 72 hours post-fertilisation, dystrophin is present in the neural tube and notochord in both wild type (**G**) and *sap* (**J**, asterisks) but lacking from muscle attachments in *sap* (arrowheads in G, **J,** transverse sections). β-dystroglycan is also localised to muscle attachments (arrowheads) and other sites (asterisks) in wild type (**H**), but unlike dystrophin is preserved at muscle attachments in *sap* (K 72 hours post-fertilisation, transverse sections). Utrophin is not detectable at muscle attachments in either wild type (**I**) or *sap* (**L** arrowheads). Anti-utrophin immunoreactivity in the epidermis provides an internal positive control (asterisks).

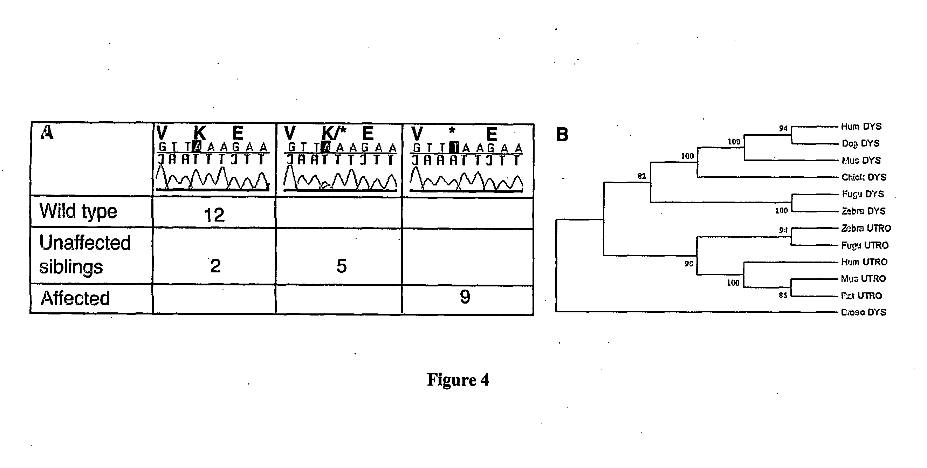

Figure 4 shows (**A**) homozygosity for the nonsense mutation (AAA→TAA) in *sap^{ta222a} dmd* exon 4 segregated exclusively with the *sap* phenotype. (**B**) Rooted tree showing dystrophin and utrophin proteins in vertebrates. The tree is rooted using *Drosophila melanogaster* dystrophin. The numbers represent the percentage of 1000 bootstrap trials that support the branch. DYS, dystrophin; UTRO, utrophin; Hum, *Homo sapiens;* Dog, *Canis familiaris;* Mus, *Mus musculus;* Chick, *Gallus gallus;* Fugu, *Fugu rubripes;* Zebra, *Danio rerio;* Rat, *Rattus norvegicus.* Proteins accession numbers: XP_081212 NP_000100 097592 NP_031894 CAA31746 NP_009055 CAA58496. The Fugu sequences are manually corrected GENSCAN predictions from genomic scaffolds (www.jgi.doe.gov/fugu, Scaffold 234). The zebrafish utrophin sequence is predicted from the zebrafish genome project. The tree has been made from partial sequences corresponding to the zebrafish protein.

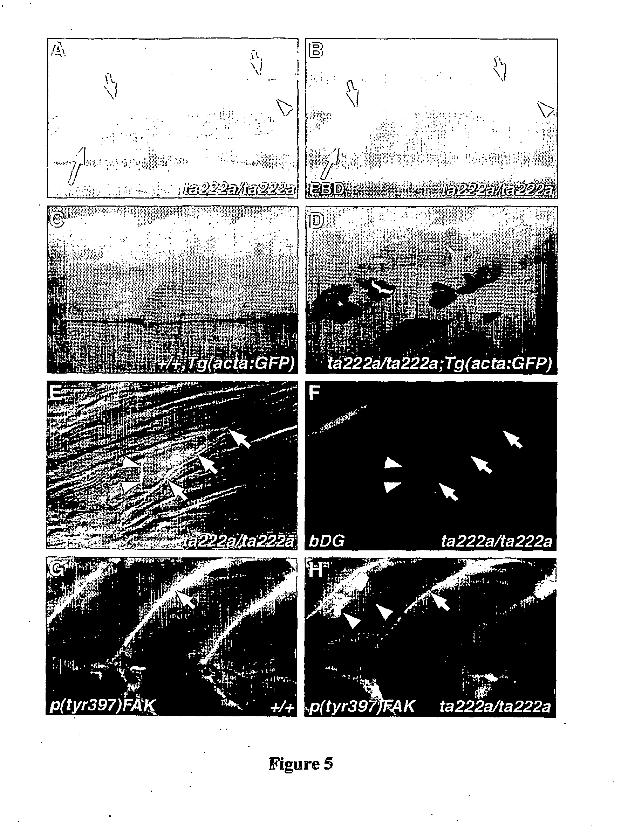

Figure 5 shows *in vivo* observation of muscle attachment failure and molecular analysis of detached free ends. A single fibre (**A, B** short arrows) viewed *in vivo* in the process of detaching myosepta, under differential interference contrast (**A,** lateral view, 5 days post-fertilisation) and labelled with EBD (**B**). A gap is visible between the separating posterior end of the fibre (right short arrow) and the myoseptum (arrowhead). A narrowed retraction zone has formed where the contractile apparatus has withdrawn from the centre of the fibre (between the short arrows). The anterior end of the fibre (left short arrow) is partly obscured by a second dye-positive detached cell (long arrow). Confocal microscopy of GFP revealed this example of a free end of a single detached fibre **(D,** bracket, lateral view, 6 days post-fertilisation). Stacked confocal images allow tracing of individual fibres to their insertion points at the muscle attachments. Fibres within *sap* homozygotes **(D)** exhibit a club-like or faceted appearance at their newly detached membranes, not evident in wild type embryos (**C**). β-DG protein at the muscle attachments is not retained in the fibre membranes once detachment occurs. Two neighbouring detached and retracted mutant cells visible under DIC (arrowheads in **E**, 72 hours post-fertilisation, lateral view) are still attached to their posterior (right) myoseptum (arrows in **E, F**). Labelling with anti-β-DG (**F**) shows that this integral membrane protein has been lost from their free (left) ends upon detachment, possibly maintaining its binding to α-dystroglycan and laminin at the myoseptum. Phosphorylated focal adhesion kinase is enriched in terminal cytoplasm at muscle attachments (arrows in **G, H,** lateral confocal images, 72 hours post-fertilisation). Unlike β-DG, however, p(tyr397)FAK remains visibly localised to the free end of detached cells, indicating the retention of terminal cytoplasm by detached and retracted fibres (arrowheads in **H).**

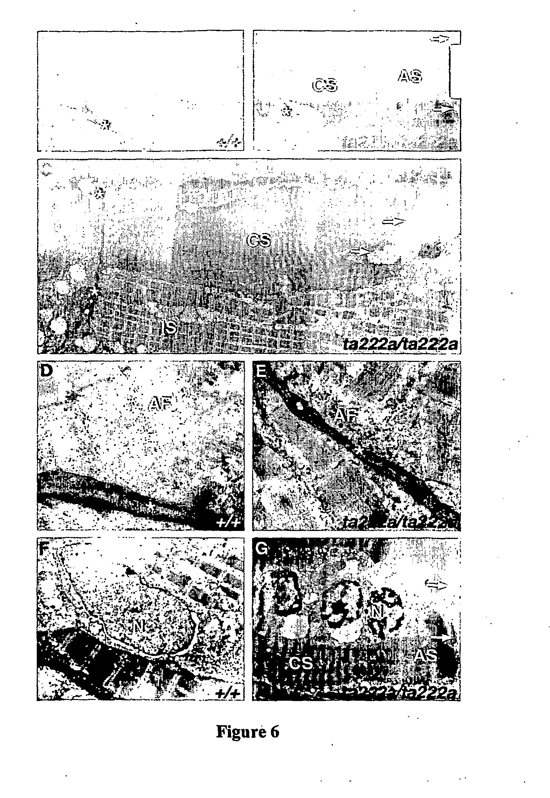

Figure 6 is electron microscopy showing that wild type embryonic myofibrils align to form a regular sarcomeric array that attaches obliquely to the myosepta (asterisks) (**A**). In *sap* homozygotes, fibres showing detached ends (arrows in **B, C, G**) and shortening of both the entire fibre and the sarcomeres, are visible. In these cells, the separation and regularity of sarcomeric banding is greatly reduced or collapsed compared to that in intact neighbouring cells, and absent in some places (**B, C**). Actin filaments (AF) run longitudinally from the terminal sarcomeres to the vertical myoseptum in both wild type and intact *sap* mutant muscle fibres (**D, E**). Nuclear changes were also followed by electron microscopy in order to examine whether detachment precedes or follows cell death in *sapje* mutants. Nuclear condensations indicative of apoptosis were only present in detached mutant fibres (**G**) but were not observed in either intact mutant or wild-type (**F**) fibres, demonstrating that detachment is not a secondary process resulting from apoptosis of muscle fibres. 4 days post-fertilisation, parasagittal sections. CS = collapsed sarcomeres, IS = intact sarcomeres, AS = absent sarcomeres, N = nucleus. Magnifications: A, F = 6000x; B, C, G = 3000x; D, E = 22000x.

### Mode(s) for Currying Out the Invention

### METHODS

### Immunohistochemistry

We carried out immunohistochemistry as previously described (Macdonald, R. et* al. The Pax protein Noi is required for commissural axon pathway formation in the rostral forebrain. Development 124, 2397-2408 (1997)). Anti-dystrophin MANDRA1 (Sigma) was diluted 1:1000. Anti-β-DG (Novocastra) was diluted 1:10. Anti-MyHC A4.1025 (DSHB, University of lowa) was used diluted 1:400. Appropriate Alexa-dye-labelled secondary antibodies (Molecular Probes) were used. Alexa-594-α-Bungarotoxin and DAPI (Molecular Probes) were diluted 1:1000.

### In situ hybridisation

We carried out *in situ* hybridisation as previously described (Macdonald, R. et al. The Pax protein Noi is required for commissural axon pathway formation in the rostral forebrain. Development 124, 2397-2408 (1997)).

### Evans Blue Dye labelling

Evans Blue Dye (Sigma) was injected at 0.1 mg/ml⁻¹ directly into the pericardium of anaesthetised embryos, which were examined and photographed 4-6 hours later.

### Confocal microscopy

We used a Zeiss LSM 510 confocal microscope and Zeiss software.

### Fish strains and maintenance

Complementation analysis of the dystrophic mutant class was carried out on mutations obtained from the Tübingen Stock Centre. In this analysis *sapje*^{tm90c} and a second unnamed mutation, *ta222a* failed to complement. Subsequent analysis has shown that extant stocks of both strains held in Edinburgh and Tübingen carry identical point mutations, suggesting that these different alleles may result from a single founder mutation within the original mutant screen. We therefore refer here to one mutation, *sapje*^{tm90c}, although analysis was carried out on both strains.

### Mapping

*dmd* was previously mapped using the LN54 radiation panel to between Z5508 and Z5085 (Bolanos-Jimenez, F. et al. Molecular cloning and characterization of dystrophin and Dp71, two products of the Duchenne Muscular Dystrophy gene, in zebrafish. Gene 274, 217-226 (2001)). We established linkage between *sap* and *dmd* using Z5508 on individual embryos from a mapping cross versus the Wik strain.

### Cloning of zebrafish dmd and identification of point mutation

Initial identification of zebrafish sequences was carried out by comparing human dystrophin Dp427m to the zebrafish whole genome shotgun. Exonic sequences were used to design primers for PCR from cDNA pools. We extracted mRNA from wild-type and mutant embryos using DynaBeads (Dynal) and made cDNA pools (Roche). PCR products were cloned in pGem-T (Promega), sequenced, and assembled and compared using Sequencher.

### Morpholino

We purchased morpholino antisense oligonucleotide MO1 from GeneTools. We prepared and injected solutions as described (Xu, X. et al. Cardiomyopathy in zebrafish due to mutation in an alternatively spliced exon of titin. Nat. Genet. 30, 205-209 (2002)).

### Sequence analysis

Sequences were aligned using CLUSTALW (v1.82 with default settings) (Higgins, D. G., Thompson, J. D. & Gibson, T. J. Using CLUSTALW for multiple sequence alignments. Methods in Enzymology 266, 383-402 (1996)). Positions in alignments containing gaps were omitted from subsequent analyses. All phylogenetic trees were constructed by the neighbour-joining method (Saitou, N. & Nei, M. The neighbor-joining method: a new method for reconstructing phylogenetic trees. Molecular Biology of Evolution 4, 406-425 (1987)) based on the proportion of amino acid sites at which sequences compared were different. The reliability of each interior branch of a given topology was assessed using the bootstrap interior branch test with 1000 bootstrap replications (Dopazo, J. Estimating errors and confidence intervals for branch lengths in phylogenetic trees by a bootstrap approach. Journal of Molecular Evolution 38, 300-304 (1994)). Phylogenetic trees were constructed using MEGA (Kumar, S., Tamura, K., Kakobsen, I. B. and Nei, M. MEGA2: molecular evolutionary genetics analysis software. Bioinformatics 17, 1244-1245 (2001)) (v2.1; http://www.megasoftware.net/) and alignments were examined and formatted in GeneDoc (v2.6.02;http://www.psc.edu/biomed/genedoc/). The *Fugu* data has been provided freely by the Fugu Genome Consortium for use in this publication/correspondence only.

Genbank Accession numbers: *Danio rerio dystrophin (dmd)* 5' sequence is AY162403 and *Fugu rubripes dystrophin* predicted 5' sequence is BK000643.

### Fish maintenance

Zebrafish strains were grown and breed as previously described in "The zebrafish book, A guide for the laboratory use of zebrafish (Danio rerio). M. Westerfield ED University of Oregon press.

### Fish Model

Zebrafish have been used mainly as a developmental biological model, studying the role of different genes during the early stages of their development. Mutations have been generated by ENU mutatgenesis and the genes underlying these mutations identified by cloning strategies. More recently, fish have become a mainstream model for medical research with the realisation that many aspects of their physiology and the genetic control of biological process are similar to mammals. The *sap* mutant, however, remains the first example of a human muscular dystrophy to be modelled in zebrafish. The ability to produce large numbers of progeny in a relative short period of time, the fact that you can breed zebrafish at high density and the access to every stage of their development, lends zebrafish to high through put screening rationales.

In term of finding agents or genes that modify the *sap* phenotype, we envisage two different types of experimental design. Firstly genetic, second site suppressor and enhancer screens where the genome is mutated to identify linked genes which can suppress or enhance the dystrophic phenotype, which themselves represent targets for therapy. A second approach is to add chemical libraries to the growing mutant zebrafish larvae to determine chemicals that suppress the mutant phenotype and there by compensate for the loss of dystrophin. The identified molecules would then be candidates for drugs to treat human muscular dystrophies and cardiomyopathies.

### RESULTS

Myotomal lesions are first evident in homozygous *sap* mutant embryos at 36 hours post fertilisation (hpf), after an initial period of muscle development within which muscle fibres elongate and striate normally within the myotome (Figure 1A, B). From this time, lesions in *sap* homozygous mutant embryos progressively accumulate until death occurs at larval stages (median =31 days post fertilisation (dpf), n=25).
Histological analysis revealed that fibres associated with lesions had detached from the vertical myosepta at the somite boundaries and are dramatically shortened (Figure 1C, D). An examination of the expression of myosin heavy chain (MyHC) within *sap* homozygous mutants confirms that fibre differentiation appears to occur in the correct spatial and temporal sequence within these embryos. However, lesions are associated with fibre loss, producing MyHC negative gaps within the differentiated myotome (Figure 1E, F). The extent to which damage is sustained by individual mutant somites was examined using an α-actin::EGFP transgene (Higashijima, S., Okamoto, H., Ueno, N., Hotta, Y. & Eguchi, G. High-frequency generation of transgenic zebrafish which reliably express GFP in whole muscles or the whole body by using promoters of zebrafish origin. Dev Biol 192, 289-299 (1997)) under confocal microscopy. This revealed extensive fibre damage in some somites, whilst neighbouring areas remained almost unaffected, suggesting that variable factors such as motor activity may contribute to degeneration (Figure 1 G, H).

The similarity of the *sap* phenotype to muscular dystrophies led us to identify the zebrafish *dystrophin (dmd)* gene, revealing an intimate association of its expression with fibre ends. *dmd* mRNA becomes localised intracellularly towards somite boundaries before muscle fibre differentiation occurs and remains so throughout development (Figure 2A, B), being subsequently concentrated at the ends of muscle fibres (Bolanos-Jimenez, F. et al. Molecular cloning and characterization of dystrophin and Dp71, two products of the Duchenne Muscular Dystrophy gene, in zebrafish. Gene 274, 217-226 (2001)). This pattern is preserved in *sap.* Dystrophin protein also localises in somitic muscle to the ends of fibres at the point where they attach to the vertical myoseptum (Figure 2C, D), which is a fibrous, cell free, sheet of extracellular matrix containing Tenascin-C (Figure 2D) (Roy, M. N., Prince, V. E. & Ho, R. K. Heat shock produces periodic somitic disturbances in the zebrafish embryo. Mech Dev 85, 27-34 (1999)). This localisation persists until at least 6 dpf. Dystrophin was not detectable at the embryonic sarcolemma before 6 dpf, however it is known to be present within the adult sarcolemma (Chambers, S. P. et al. Dystrophin in adult zebrafish muscle. Biochem. Biophys. Res. Commun. 286, 478-483 (2001)). Embryonically, dystrophin is detectable within the myotome, but colocalises entirely with neuromuscular junctions (NMJ, Figure 2E-G). Thus, the major site of embryonic dystrophin expression is the ends of muscle fibres where they attach via the vertical myoseptum to the fibres of the next somite, generating myomuscular junctions (MMJs) (Waterman, R. E. Development of the lateral musculature in the teleost, Brachydanio rerio: a fine structural study. Am. J Anat. 125, 457-493 (1969)).

*sap* mutants lack dystrophin immunoreactivity at muscle fibre ends, but retain later expression at sites outside the somites (Figure 3A, B, C, D), suggesting a selective loss of isoforms. This observation is consistent with the *sap* phenotype resulting from damage specific to the boundary regions of somitic muscle. Localisation of the transmembrane β-chain of the dystrophin-binding laminin receptor, dystroglycan (β-DG), which is required for muscle differentiation, is normal in *sap* (Figure 3I, J), indicating that dystrophin is not required for this process (Henry, M. D. & Campbell, K. P. Dystroglycan inside and out. Curr. Opin. Cell Biol. 11, 602-607 (1999); Parsons, M. J., Campos, I., Hirst, E. M. & Stemple, D. L. Removal of dystroglycan causes severe muscular dystrophy in zebrafish embryos. Development 129, 3505-3512 (2002)). To investigate the effects of *sap* on membrane integrity at the MMJ, we injected Evans Blue Dye (EBD), which labels cells with compromised membranes (Hamer, P. W., McGeachie, J. M., Davies, M. J. & Grounds, M. D. Evans Blue Dye as an in vivo marker of myofibre damage: optimising parameters for detecting initial myofibre membrane permeability. J. Anal. 200, 69-79 (2002)). EBD labels many detached fibres in *sap* somites (Figure 3F, G), and a few fibres that are still attached at both ends. Such labelling indicated that there was loss of membrane integrity when fibres detach their ends from myosepta. Loss of dystrophin, retention of dystroglycan, and membrane damage all occur in Duchenne muscular dystrophy (Spence, H .J., Chen, Y. J. & Winder, S. J. Muscular dystrophies, the cytoskeleton and cell adhesion. BioEssays 24, 542-552 (2002)), making *dmd* a strong candidate for *sap.*

Comparing the human dystrophin protein to the zebrafish whole genome shotgun reads using BLAST identified fragments of a single gene, which we subsequently joined by PCR to recover cDNA sequences representing nearly the complete open reading frame. Comparisons of the N-terminal region with other species, including a predicted *Fugu* protein which we identified (Figure 3K, L), confirmed earlier findings that zebrafish *dmd* is the true orthologue of the human *DMD* gene (Chambers, S. P, et al. Dystrophin in adult zebrafish muscle. Biochem. Biophys. Res. Commun. 286, 478-483 (2001)).

*dmd* was previously mapped to Linkage Group 1 (LG1), and we used markers at this position to test for linkage to *sapje^{tm90c}.* This revealed close linkage, and we therefore examined *sapje^{tm90c}* for mutations in *dmd* (Figure 3K, L). The absence of dystrophin C-terminal immunoreactivity from *sap* muscle but not from other sites suggested that sapje^{tm90c} might be a mutation in an exon of *dmd* retained exclusively at embryonic stages in muscle-specific isoforms. This and the large size of the human orthologue led us to initiate mutation detection within 5' exons of the zebrafish gene homologous to those present in Dp427m, the isoform of dystrophin that predominates in mammalian muscle. Within exon 4 of *dmd* from *sapje*^{tm90c} we found an A→T transversion causing a nonsense mutation within the first calponin homology (CH) domain, which segregated in the homozygous state exclusively with the *sap* phenotype (Figure 3K, L). By comparison, a nonsense mutation in exon 4 of human *DMD* causes DMD, indicating that this exon is essential in human muscle (Sitnik, R. et al. Novel point mutations in the dystrophin gene. Hum. Mutat. 10, 217-222 (1997)). To conform that this mutation causes the *sap* phenotype we injected an antisense morpholino oligonucleotide (MO1), targeted to overlap the boundary of zebrafish dmd exon 6 and its downstream intron, at the 1-cell stage. This was predicted to produce exon-skipping (Xu, X. et al. Cardiomyopathy in zebrafish due to mutation in an alternatively spliced exon of titin. Nat. Genet. 30, 205-209 (2002)) from exon 5 to exon 7, resulting in a frameshift and premature termination within exon 7, and its effectiveness was evidenced by the absence of dystrophin C-terminal immunoreactivity in injected embryos (Figure 3C). MO1 injections phenocopied *sap* (46/159, 29%), causing somitic EBD uptake and lesions that were never seen among embryos injected with control morpholinos (Figure 3C, H). Thus, we conclude that the mutation in *dmd* causes the sapje^{tm90c} phenotype.

Partial alignment of zebrafish (Dr_dys), predicted *Fugu rubripes* (Fr_predict), human (Hs_dys) and chicken (Gg_dys) dystrophin proteins is shown in Figure **4A**. The region shown includes two N-terminal calponin homology domains (CH, underlined), and is encoded by exons 2 to 7. The termination site of the *sap*^{tm90c} form of zebrafish dystrophin is marked by an asterisk. Estimated positions of exon boundaries are marked by green arrowheads, except that between exons 6 and 7 (red arrowhead) against which morpholino MO1 was directed.

Rooted tree (Figure 4B) showing dystrophin and utrophin proteins in vertebrates. The tree is rooted using *Drosophila melanogaster* dystrophin. The numbers on each arm, represent the percentage of 1000 bootstrap trials that support the branch. DYS, dystrophin; UTRO, utrophin, Hum, *Homo sapiens;* Dog, *Canis familiaris;* Mus, *Mus musculus;* Chick, *Gallus gallus;* Fugu, *Fugu rubripes; Zebra, Danio rerio;* Rat, *Rattus norvegicus.* Proteins accession numbers: Drosophila dystrophin, XP_081212; Human dystrophin, NP_000100; Dog dystrophin, 097592; Mouse dystrophin, NP_031894; Chicken dystrophin, CAA31746, Human utrophin, NP_009055; Mouse utrophin, CAA58496. The Fugu sequence is a manually corrected GENSCAN prediction from a genomic scaffold (www.jgi.doe.gov/fugu, Scaffold 234). The tree has been made from partial sequences corresponding to the zebrafish protein.

In 28 individual embryos tested, the nonsense mutation identified (AAA→TAA) in *dmd* was found to segregate in the homozygous state exclusively with the *sap*^{tm90c} phenotype.

In order to understand the cellular basis for muscles degeneration present within *sap* homezygotes, we continuously monitored mutant somites using both light microscopy and EBD-labelling. EBD labels fibres prior to retraction and we were consequently able to observe single mutant fibres *in vivo* detaching from myosepta (Figure 5A, B). This detachment is characterised by a progressive stretching and tearing from the myoseptum of fibre end membranes and a consequent failure of attachment at the MMJ. Consistent with these observations, utilising three-dimensional confocal microscopy to trace newly detached fibres along the entirety of their length revealed morphological abnormalities specifically associated with the free fibre ends. Detached fibres often possessed a novel club-like or multifaceted appearance. Thus, the degeneration of muscle tissue in *sap* mutants occurs through failure of the MMJ with associated loss of membrane integrity. Our analysis of the phenotype of homozygous *sap* mutant embryos represents the first genetic evidence that dystrophin is required for the formation of stable attachments at the end of vertebrate muscle fibres.

In order to determine precisely where detachment and membrane damage occur in *sap,* we examined the MMJ of *sap* mutants using immunohistochemistry to detect proteins normally localised either within the terminal membrane or immediately intracellular to it. In *sap* mutants the transmembrane protein β-dystroglycan remains associated with the myoseptum when fibres detach, consistent with failure at the level of dystrophin, which lies immediately intracellular to the sarcolemma at the MMJ (Figure 5E, F). However, focal adhesion kinase (FAK), an integrin-associated cytoplasmic protein enriched at the embryonic somite boundary, is retained in free the ends of detached fibres, also indicating that failure occurs near the plane of the sarcolemma. Therefore, membrane integrity is lost on the inner side of the plasma membrane, consistent with a structural failure of dystrophin linkage between the actin cytoskeleton and the plasma membrane causing the observed detachments.

The pathology of Duchenne and other muscular dystrophies has been thought to result primarily from the loss of proteins of the DGC from the sarcolemma, leading to a loss of mechanical support between muscle fibres and membrane tearing during contraction (Spence, H .J., Chen, Y. J. & Winder, S. J. Muscular dystrophies, the cytoskeleton and cell adhesion. BioEssays 24, 542-552 (2002)). However, dystrophin-deficient *mdx* mice recover from an acute phase of muscle fibre necrosis without sarcolemmal damage or permanent debilitation, perhaps because of efficient regeneration or compensation by the related protein utrophin (Cullen, M. J. & Jaros, E. Ultrastructure of the skeletal muscle in the X chromosome-linked dystrophic (mdx) mouse. Comparison with Duchenne muscular dystrophy. Acta Neuropathol. (Berl) 77, 69-81 (1988); Sicinski, P. et al. The molecular basis of muscular dystrophy in the mdx mouse: a point mutation. Science 244, 1578-1580 (1989); Deconinck, A. E. et al. Utrophin-dystrophin-deficient mice as a model for Duchenne muscular dystrophy. Cell 90, 717-727 (1997); Grady, R. M. et. al. Skeletal and cardiac myopathies in mice lacking utrophin and dystrophin: a model for Duchenne muscular dystrophy. Cell 90, 729-738 (1997)). Another possible reason for the mild phenotype of *mdx* mice could be that dystrophin is required at membrane specialisations such as muscle end attachments in humans but less so in mouse fibres.

Dystrophin is enriched both at mammalian myotendinous junctions (MTJs) and within large mammalian muscles at MMJs attaching fibres in series. Mammalian muscles more than a few centimetres long are arranged either as mingled overlapping fibres joined end-to end and end-to-side at intrafascicular fibre terminations (IFTs) (Paul, A. C., Sheard, P. W., Kaufman, S. J. and Duxson, M. J. Localization of alpha7 integrins and dystrophin suggests potential for both lateral and longitudinal transmission of tension in large mammalian muscles. Cell Tissue Res. 308, 255-265 (2002); Snobl, D., Binaghi, L. E. & Zenker, W. Microarchitecture and innervation of the human latissimus dorsi muscle. J. Reconstr. Microsurg. 14, 171-177 (1998)) or as blocks of non-overlapping fibres separated by fibrous sheets called tendinous intersections (Tls) (Hijikata, T. & Ishikawa, H. Functional morphology of serially linked skeletal muscle fibers. Acta Anat. (Basel) 159, 99-107 (1997)). IFTs are known to contain dystrophin, while Tls are of unknown composition but bear a striking structural resemblance to the dystrophin-dependent attachments we describe here, raising the possibility of molecular similarities in formation and function. In the light of our data, the presence of dystrophin at fibre end attachments in larger mammalian muscles raises the possibility that their failure contributes to the pathology of DMD. A potential mechanism for this is that the detachment of end-to-side junctions could result in the characteristic membrane tearing found along the length of fibres in DMD and subsequent degeneration. If failure at such sites were a primary pathological mechanism in dystrophinopathies, their differential deployment and failure would account for both the apparent correlation between the size of a mammal and the severity of its phenotype, and the variation in pathology between muscle groups within either DMD patients or *mdx* mice.

Given the highly penetrant nature of the model according to the present invention and the unique aspects of zebrafish biology, the present inventors believe the model gives a hither-too unavailable chance to screen for genes and molecules that will alleviate the dystrophic pathology. The present inventors have found that zebrafish are particularly well suited to the application of chemical libraries or small molecules and the use of combinatorial chemistry for drug discoveries. Chemicals can be screened in a high through put manner by direct application to the culture medium in which *sap* mutant fish are raised, or by direct injection into the embryo. Similarly mutation can be identified which suppress the dystrophic phenotype by mutagenesis. Existing pharmacological strategies for muscular dystrophy centre around increasing muscle mass, either through the use of steroids or agents such as creatine but the side affects of such agents can be debilitating and the long term benefits are questionable. Emphasis has also been placed trialing drugs that maintain calcium homeostasis, which has been advanced as underlying the pathology of dystrophies. Furthermore, drugs that inhibit cellular calcium release have shown some promise as treatments. Finally an important area has been treating the inflammation that occurs in models of human dystrophies and clinical trials using mast-cell stabilisers are currently underway. All these agents could be added or trialed initially in the present dystrophic fish model. It should be noted, however, that none of these treatments are designed to treat the route cause of the disease, the loss of dystrophin. We hope that by employing a non-biased screening rationale according to the present invention, we will be able to prevent the primary pathological response in dystrophic and cardiomyopathy muscle.

We demonstrate here for the first time that dystrophin is required for the stability of vertebrate muscle attachments and that muscle detachment is the primary cause of degeneration in a fish model of a muscular dystrophy or cardiomyopathy. It is therefore possible that *sap* mutants provide a model for the novel pathological mechanism of junctional failure that could have been hitherto overlooked in DMD and other muscular dystrophies or cardiomyopathy. These results suggest that the zebrafish *sap* mutant is an ideal model system for understanding the role of the dystrophin complex at muscle attachments and their failure in muscular dystrophies, or cardiomyopathy and for beginning to approach the question of relevant therapies.

### SUMMARY

The extemally-developing, transparent zebrafish embryo allows inspection of living muscle and the isolation of mutations affecting its formation and maintenance. A class of mutations has been identified in which normal differentiation of skeletal muscle is followed by degeneration reminiscent of human muscular dystrophies. Here we show that one of these, *sapje (sap),* disrupts the zebraflsh orthologue of the human *dysfrophin* gene, mutations of which result in Duchenne and Becker muscular dystrophies (DMD, BMD). In mammals, dystrophin is required to link the actin cytoskeleton with the extacellular matrix between muscle fibres. It is thought that its loss from the dystrophin glycoprotein complex (DGC) in skeletal muscle of DMD patients weakens lateral inter-fibre linkages allowing tearing of the sarcolemma, or cell membrane, along the length of fibres. Surprisingly, we find that within the zebrafish embryo, dystrophin localises exclusively to the ends of muscle fibres at a site that we have defined as the embryonic myomuscular junction (MMJ). Furthermore, we show that the dystrophic phenotype of *sap* mutants is characterised by the detachment of fibre ends from this site and loss of fibre membrane integrity. Although a functional role for dystrophin in maintaining junctional integrity of muscle fibres has long been postulated, direct evidence for such a role has been lacking. This mutation may therefore provide a model for a novel pathological mechanism in DMD, other dystrophies and cardiomyopathy.

It will be appreciated by persons skilled in the art that numerous variations and/or modifications may be made to the invention as shown in the specific embodiments. The present embodiments are, therefore, to be considered in all respects as illustrative and not restrictive.

## Claims

1. An isolated zebrafish genetic strain having a dystrophin mutant phenotype resulting from a mutation within the zebrafish dystrophin gene, wherein the zebrafish has a *sapje (sap)* mutant phenotype.

2. The zebrafish according to claim 1 having a mutation selected from the group consisting of sapje tm90c, tj7, ta222a, and combinations thereof.

3. The zebrafish according to claim 2 having the sapje tm90c mutation.

4. A fish model of mammalian muscular dystrophy comprising an isolated zebrafish according to any one of claims 1 to 3 or progeny, fry, or gametes thereof.

5. The fish model according to claim 4 wherein the mammalian muscular dystrophy is human muscular dystrophy.

6. A method for screening an agent having potential activity on muscular dystrophy or monitoring or testing the effect of an agent having activity on muscular dystrophy or cardiomyopathy comprising:
(a) providing a fish model according to claim 4 or 5;
(b) exposing the zebrafish to an agent; and
(c) determining any effect of the agent on a genetic or physical characteristic of the zebrafish or its progeny.

7. The method according to claim 6 wherein the agent is a drug candidate, chemical, compound, nucleic acid, or mixture thereof.

8. The method according to claim 6 or 7 wherein the fish is exposed to the agent by addition to fish raising media.

9. The method according to any one of claims 6 to 8 wherein the effect is determined by a visual or light microscopic technique including techniques that utilise transgenic reporter gene expression to monitor muscle integrity.

10. The method according to any one of claims 6 to 8 wherein the effect is determined by simple optical inspection of living muscle tissue, birefringency of muscle tissue using polarised light, use of Green fluorescent protein transgenic lines driven by muscle specific promoter(s), use of immunohistochemistry, use of antibodies directed against muscle specific epitopes or *in situ* hybridisation for muscle specific gene expression.

## Patentansprüche

1. Isolierter genetischer Zebrafischstamm mit einem Dystrophin-mutierten Phänotyp, der aus einer Mutation innerhalb des Zebrafisch-Dystrophingens resultiert, wobei der Zebrafisch einen *sapje*-(*sap*)-mutierten Phänotyp hat.

2. Zebrafisch nach Anspruch 1 mit einer Mutation ausgewählt aus der Gruppe bestehend aus sapje tm90c, tj7, ta222a und Kombinationen davon.

3. Zebrafisch nach Anspruch 2 mit der sapje-tm90c-Mutation.

4. Fischmodell der Säugetier-Muskeldystrophie umfassend einen isolierten Zebrafisch gemäß einem beliebigen der Ansprüche 1 bis 3 oder Nachkommen, Brut oder Keimzellen davon.

5. Fischmodell nach Anspruch 4, wobei die Säugetier-Muskeldystrophie humane Muskeldystrophie ist.

6. Verfahren zum Screenen eines Mittels mit potentieller Wirkung gegenüber Muskeldystrophie oder Überwachen oder Testen des Effekts eines Mittels mit Wirkung gegenüber Muskeldystrophie oder Kardiomyopathie umfassend:
(a) Bereitstellen eines Fischmodells gemäß Anspruch 4 oder 5;
(b) Aussetzen des Zebrafisches einem Mittel; und
(c) Bestimmen des Effekts, den das Mittel auf ein genetisches oder äußerliches Merkmal des Zebrafisches oder seiner Nachkommen hat.

7. Verfahren gemäß Anspruch 6, wobei das Mittel ein Wirkstoffkandidat, eine Chemikalie, Verbindung, Nukleinsäure oder eine Mischung davon ist.

8. Verfahren gemäß Anspruch 6 oder 7, wobei der Fisch dem Mittel durch Hinzufügen zu dem Fischnährmedium ausgesetzt wird.

9. Verfahren gemäß einem beliebigen der Ansprüche 6 bis 8, wobei der Effekt durch eine visuelle oder lichtmikroskopische Technik bestimmt wird, einschließlich Techniken, die transgene Reporter-Genexpression zum Überwachen der Muskelintegrität verwenden.

10. Verfahren gemäß einem beliebigen der Ansprüche 6 bis 8, wobei der Effekt durch einfache optische Inspektion von lebendem Muskelgewebe, Doppelbrechung des Muskelgewebes unter Verwendung von polarisiertem Licht, Verwendung von transgenen Linien mit grünfluoreszierendem Protein unter der Kontrolle eines/von muskelspezifischen Promotor(s/en), Verwendung von Immunhistochemie, Verwendung von Antikörpern gerichtet gegen muskelspezifische Epitope oder in-situ-Hybridisierung für muskelspezifische Genexpression.

## Revendications

1. Souche génétique de poisson-zèbre isolé ayant un phénotype mutant de dystrophine résultant d'une mutation dans le gène de la dystrophine de poisson-zèbre, dans laquelle le poisson-zèbre possède un phénotype mutant *sapje* (*sap*).

2. Poisson-zèbre selon la revendication 1, possédant une mutation choisie dans le groupe constitué de sapje tm90c, tj7, ta222a, et leurs combinaisons.

3. Poisson-zèbre selon la revendication 2, possédant la mutation sapje tm90c.

4. Modèle poisson de dystrophie musculaire de mammifère comprenant un poisson-zèbre isolé selon l'une quelconque des revendications 1 à 3 ou bien une descendance, un alevin, ou des gamètes de celui-ci.

5. Modèle poisson selon la revendication 4, dans lequel la dystrophie musculaire de mammifère est une dystrophie musculaire humaine.

6. Procédé pour cribler un agent ayant une activité potentielle sur une dystrophie musculaire ou bien pour surveiller ou tester l'effet d'un agent ayant une activité sur une dystrophie musculaire ou une cardiomyopathie comprenant :
(a) la fourniture d'un modèle poisson selon la revendication 4 ou 5 ;
(b) l'exposition du poisson-zèbre à un agent ; et
(c) la détermination de tout effet de l'agent sur une caractéristique génétique ou physique du poisson-zèbre ou de sa descendance.

7. Procédé selon la revendication 6, dans lequel l'agent est un candidat médicamenteux, un composé chimique, un acide nucléique, ou leur mélange.

8. Procédé selon la revendication 6 ou 7, dans lequel le poisson est exposé à l'agent par addition au milieu d'élevage de poissons.

9. Procédé selon l'une quelconque des revendications 6 à 8, dans lequel l'effet est déterminé à l'aide d'une technique microscopique visuelle ou optique incluant des techniques qui utilisent une expression de gène rapporteur transgénique afin de surveiller l'intégrité du muscle.

10. Procédé selon l'une quelconque des revendications 6 à 8, dans lequel l'effet est déterminé à l'aide d'une simple inspection à l'oeil de tissu musculaire vivant, d'une biréfringence de tissu musculaire en utilisant une lumière polarisée, d'une utilisation de lignées transgéniques de protéines fluorescentes vertes conduites par un (des) promoteur(s) spécifique(s) d'un muscle, d'une utilisation d'immunohistochimie, d'une utilisation d'anticorps dirigés contre des épitopes spécifiques d'un muscle ou d'une hybridation *in situ* pour une expression de gène spécifique d'un muscle.
